# EUROPÄISCHE PATENTSCHRIFT

(11) **EP 0 331 102 B1**
(45) Veröffentlichungstag und Bekanntmachung des Hinweises auf die Patenterteilung: **23.06.1993**
(21) Anmeldenummer: 89103480.3
(22) Anmeldetag: 27.02.1989
(51) Int. Cl.: A61K 39/00, A61K 39/17, A61K 39/39

(54) **Virusmodifizierte Tumorvakzinen für die Immuntherapie von Tumormetastasen**
Virus-modified tumour vaccine for the immunotherapy of tumour metastases
Vaccin tumoral modifié avec des virus pour l'immunothérapie des métastases tumorales

(30) Priorität: 01.03.1988 DE 3806565
(43) Veröffentlichungstag der Anmeldung: 06.09.1989
(73) Patentinhaber: Schirrmacher, Volker, Prof. Dr., D-69117 Heidelberg (DE)
(72) Erfinder: Schirrmacher, Volker, Prof. Dr., D-69117 Heidelberg (DE)
(74) Vertreter: Schüssler, Andrea, Dr.

(56) Entgegenhaltungen:
- WO-A-86/00811
- CLINICAL AND EXPERIMENTAL METASTASIS, Band 5, Nr. 2, 1987, Seiten 147-156; V. SCHIRRMACHER et al.: "Prevention of metastatic spread by postoperative immunotherapy with virally modified autologous tumor cells. II. Establishment of specific systemic anti-tumor immunity"
- BIOLOGICAL ABSTRACTS, Band 70, Nr. 5, Seite 3213, Nr. 30587, Biological Abstracts Inc., Philadelphia, US; M.D. EATON: "Autoimmunity induced by syngeneic splenocyte membranes carrying irreversibly adsorbed paramyxovirus", & INFECT. IMMUN. 27(3) 855-861. 1980
- BIOLOGICAL ABSTRACTS, Band 57, Nr. 7, 1. April 1974, Seiten 4126-4127, Nr. 38826, Philadelphia, US; M.D. EATON et al.: "Enhancement of lymphoma cell immunogenicity by infection with nononcogenic virus", & CANCER RESEARCH 33(12), 3293-3298, 1973
- CANCER IMMUNOLOGY IMMUNOTHERAPY, Band 28, Nr. 1, 1988, Seiten 22-28, Springer-Verlag; H. SCHILD et al.: "Modification of tumor cells by a low dose of Newcastle disease virus. II. Augmented tumor-specific T cell response as a result of CD4+ and CD8+ immune T cell cooperation"

## Beschreibung

Die Erfindung betrifft virus-modifizierte, tumorspezifische Vakzine, bestehend aus durch Bestrahlung inaktivierten entsprechenden Tumorzellen, inkubiert mit NDV-Virus unter sterilen Bedingungen, in serumfreinem Medium sowie Verfahren zur Herstellung der Vakzine.

Ein Problem bei der operativen Therapie von malignen Tumoren besteht auch nach weitgehender Entfernung des Primärtumors in der Verhinderung der Bildung von Metastasen, die auf dem Auswachsen von zum Zeitpunkt der Operation bereits vorhandenen Mikrometastasen, oder aber der Bildung neuer Metastasen durch bei der Operation ausgesäte Tumorzellen beruht.

Neben klassischen chemotherapeutischen Ansätzen hat man in neuerer Zeit auch versucht, das körpereigene Immunsystem des Patienten so zu aktivieren, daß es durch Bildung von möglichst tumorspezifischen T-Killerzellen die Bildung von Mikrometastasen verhindert, bzw. bereits vorhandene Metastasen eliminiert. Zu diesem Zweck wurden, mit unterschiedlichem Erfolg, sogenannte Onkolysate, d.h. Lysate von Turmozellen, meist von Tumorzelllinien, zur Immunisierung des Patienten eingesetzt. Dabei ergibt sich das Problem, daß diese Zellysate, die zudem in der Regel nicht aus den eigentlichen Tumorzellen, sondern aus in Zellkultur gehaltenen Tumorzelllinien gewonnen werden, nicht ausreichend immunogen sind und deshalb nicht zu einer ausreichenden Stimulierung der Immunabwehr des Patienten führen, so daß die Metastasierung wirksam unterbunden werden kann.

Auch ist aus Clinical and Experimental Metastasis, Band 5, Nr. 2 (1987), 147 - 156 ein Maus-Tumor-Modell bekannt, das die Kombination aus einem operativen Eingriff und einer postoperativen Immuntherapie unter Verwendung von virus-modifizierten autologen Tumorzellen vorsieht. Der Ausdruck "autolog" bedeutet dabei, daß die Tumorzellen, die zur Erzeugung des Tumors in der Maus verwendet werden, daneben auch in Kultur gehalten werden und letztere dann zur Immuntherapie der Maus mit NDV-Virus modifiziert werden.

Der vorliegenden Anmeldung liegt somit die Aufgabe zugrunde, ein Immuntherapiesystem bereitzustellen, mit dem die Aktivierung des Immunsystems bei Tumorpatienten verbessert werden kann.

Es kann davon ausgegangen werden, daß tumorspezifische immune T-Lymphozyten, wenn sie vorhanden sind, nur in einer geringen Frequenz unter den Lymphozyten vorkommen, da die Antigenität und Immunogenität von Tumorantigenen im allgemeinen nur schwach ausgeprägt ist. In einem untersuchten Tiermodell beträgt beispielsweise die Häufigkeit derartiger Zellen nur eine unter 15 000 Milzlymphocyten. In tumortragenden Tieren sind diese tumorspezifischen T-Zellen im allgemeinen nicht voll aktiviert und bedürfen zusätzliche Aktivierungssignale.

Es wurde nun gefunden, daß durch eine Kombination aus einer spezifischen und einer unspezifischen Immunogenkomponente das Immunsystem des Tumorpatienten entscheidend verbessert werden kann. Die spezifische Komponente stellen dabei die autologen Tumorzellen dar, die von dem operierten Tumor durch mechanische und enzymatische Methoden isoliert werden. Die unspezifische Komponente stellt ein Virus, insbesonders Newcastle Disease Virus (NDV) dar, das zur Aktivierung einer Entzündungsreaktion beiträgt. Das NDV ist hierfür geeignet, weil es ein guter Interferon-Aktivator ist und Untersuchungen zufolge tumorspezifische T-Zellen zu aktivieren vermag. Das Virus ist zudem besonders gut verträglich.

Zur Herstellung der Vakzine wird das Virus mit bestrahlten Tumorzellen inkubiert, was zu einer Adsorption des Virus an die Tumorzellen führt. Die virusmodifizierten Tmorzellen werden durch Bestrahlung inaktiviert, so daß sie nicht als Tumoren auswachsen können, und dann als Vakzine benutzt. Durch Immunisierung mit dieser Vakzine sollen sensibilisierte tumorspezifische T-Zellen in situ selektiv aktiviert werden, und diese Zellen, die im Blut und in der Lymphe zirkulieren, für eine systemische Therapie gegen Mikrometastasen eingesetzt werden. Nach intradermaler Impfung mit einer derartigen Vakzine wird in Tumorpatienten zunächst die Auslösung einer Hautreaktion vom verzögeren Typ angestrebt. Diese für zelluläre Immunreaktionen charakteristische Reaktion zeigt an, daß ein spezifisches Antigen erkannt wurde und zu einer Entzündungsreaktion am Ort der Impfung geführt hat. Dies konnte in Experimenten bestätigt werden. Am Ort der Impfung sollen die tumorspezifischen T-Zellen aufgrund der Anwesenheit des entsprechenden Antigens konzentriert und mit Hilfe der zusäztlichen Aktivierungssignale, die durch das Virus vermittelt werden, zu zytotoxischen T-Lymphozyten aktiviert werden. Wenn die aktivierten tumorspezifischen T-Zellen danach wieder in die Zirkulation d.h. in das Blut und in das lymphatische System, gelangen, sind sie zum Auswandern in die Organe befähigt und können dort ihre Kontrollfunktion durch Erkennung von Tumorzellen mit dem gleichen Tumorantigen und deren Entfernung, ausüben.

Der wesentliche Vorteil der Vakzinen und ihrer Anwendung zur Immuntherapie von Metastasen gemäß der vorliegenden Erfindung liegt darin, daß eine Kombination von Immunogenen eingesetzt wird, mit der Folge, daß zum einen ein autologes Tumorantigen optimal präsentiert wird, zum anderen eine zweite Komponente zur Stimulierung tumorspezifischer T-Lymphozyten optimal dosiert wird. Dies geschieht unter Simulierung einer Immunreaktion, wie sie in etwa auch unter physiologischen Bedingungen bei der Abwehr einer Virusinfektion abläuft. Gegenüber Therapieformen wie adjuvanter Chemotherapie oder auch der Applikation hoher Dosen von Lymphokinen (Interferone oder Interleukin II) hat die spezifische Immuntherapie mit den virusmodifizierten Tumorvakzinen gemäß der vorliegenden Erfindung den Vorteil, daß sie ausgesprochen nebenwirkungsarm ist. Die Verwendung von autologen intaktiven Tumorzellen sollte eine Gewähr dafür bieten, daß die optimale Antigenität und Immunogenität des Tumormaterials erhalten bleibt. Das als unspezifische virale Komponente bevorzugte Newcastle-Disease-Virus (NDV) weist im Vergleich zu anderen Viren, wie etwa Influenza A Virus oder Vaccinia Virus, die auch bei Immunisierungen mit Onkolysaten Verwendung finden, die geringsten neurotropen Nebenwirkungen auf. Gleichzeitig ist das NDV im Vergleich zu den anderen Viren ein besserer Interferon-Aktivator.

Die spezifische Immuntherapie mit virusmodifizierten Tumorvakzinen gemäß der vorliegenden Erfindung konnte an verschiedenen Modellen, nämlich dem Modellsysem ESb der Maus, dem Maus-Blasenkarzinom der C57 Bl-Maus und dem "3LL Lewis Lung" Karzinom-System, experimentell erprobt und ihre Wirksamkeit nachgewiesen werden. Inzwischen liegen nun erste Ergebnisse klinischer Studien vor, die die Wirksamkeit der erfindungsgemäßen Vakzine belegen.

Gemäß DE-OS 34 32 714 wird lyophylisiertes Tumormaterial verwendet und als Adjuvanz ein Enzym, nämlich Neuraminidase (VCN) eingesetzt. Es ist aber bekannt, daß lyophylisiertes Tumormaterial eine geringere Immunogenität besitzt als intakte Zellen und außerdem handelt es sich bei dem erfindungsgemäß benutzten Virus (NDV-Virus) sicher um ein stärkeres Adjuvanz als bei dem Enzym, denn es ist bereits der Nachweis erfolgt, daß eine Reihe immunologisch relevanter Genprodukte, wie Interferon alpha, beta und Tumornekrosefaktor alpha durch das Virus induziert wrden, während derartige Aktivitäten bei dem Enzym nicht nachgewiesen werden konnten. In dem oben erwähnten ESb-Maus-Tumor-Modellsystem hat der Vergleich zwischen virusmodifizierten Tumorzellen und Neuraminidase-behandelten Zellen, die jeweils als Stimulatorzellen verwendet wurden, um tumorspezifische T-Killer-Zellen zu aktivieren, ergeben, daß nur die virusmodifizierten Zellen in der Lage waren, eine signifikant erhöhte Cytotoxizität zu induzieren, während die Neuraminidase-behandelten Zellen sich genauso wie die nicht-behandelten Zellen verhielten.

Bei den obenerwähnten klinischen Versuchen wurden Patientengruppen verglichen, die einerseits autologe Tumorvakzine (ATV) und andererseits die erfindungsgemäße mit NDV-Virus-modifizierte ATV erhielten. Es zeigte sich, daß von 27 Patienten 26 auf die Vakzinierung angesprochen haben und daß NDV einen Verstärkereffekt auf die Hautreaktionen ausübt, so daß sich die Zahl der Patienten, die gegen den eigenen Tumor reagieren, verdoppelt und auch die Hautreaktionen vergrößert werden.

Die DE-OS 29 18 927 zeigt ein Arzneipräparat zur Behandlung von Carcinomen und ein Verfahren zu dessen Herstellung, wobei das Präparat Carcinomzellen enthält, die mit einem Cytostatikum gegenüber dem die Carcinomzellen empfindlich sind, abgeschwächt worden ist. Es wird also überhaupt kein immunologisches Adjuvanz oder Immunstimulanz verwendet, statt dessen werden mit Cytostatika behandelt lebende autologe Tumorzellen verwendet, die offenbar nicht noch anderweitig inaktiviert wurden. Hier besteht natürlich grundsätzlich das Risiko, daß theoretisch überlebende oder Cytostatika resistente Tumorzellen bei den applizierten Zellzahlen auswachsen und Tumoren bilden könnten. Daß tatsächlich immunologische Wirts-Reaktionen gegen den Tumor aktiviert werden, ist nicht ersichtlich. Der Hauptvorteil dieses Verfahrens der DE-OS 29 18 927 dürfte darin bestehen, daß nachträglich eine bessere Verträglichkeit für die Chemotherapie zu bestehen schein. Dies kann aber mit der Erfindung nicht verglichen werden.

Das Newcastle Disease Virus (NDV) eignet sich als unspezifische Komponente der Vakzine besonders, da es neben sehr guter Expression viraler Antigene auf der Zellmembran auch ein ausgezeichneter Interferon-Inducer ist. Das Virus wird im Hüherei vermehrt und vor der Inokulation in den Patienten durch Bestrahlung inaktiviert. Bezüglich der Anwendung beim Menschen ist das NDV besonders geeignet. NDV ist ein Paramyxovirus (Myxovirus avium multiforme) und der Erreger der atypischen Geflügelpest. Der Virus ist für den Menschen nur sehr wenig pathogen. Nach Infektion entwickelt sich nur bei einigen Menschen nach ein bis vier Tagen eine einseitige Konjunktivites, die sich in der Regel nach drei bis fünf Tagen, selten nach zwei bis drei Wochen zurückbildet. Bei 50% der Patienten kann es zu einer präauriculären Lymphadenitis kommen. Gelegentlich treten allgemeines Krankheitsgefühl mit Kopfschmerzen und Temperatursteigerung wie bei einem grippalen Infekt, sowie Tracheitis, Pharyngitis und Stomatitis auf. Die sehr seltene Virus-Pneumonie heilt nach fünf bis sechs Tagen aus. Zentralnervöse Störungen sowie eine haemolytische Anämie können vorkommen. In früheren Studien mit NDV-Onkolysaten von Melanompatienten wurden keine Nebenwirkungen bei der Anwendung am Menschen beobachtet.

Die Wirkungen des NDV auf das Immunsystem, sowie seine Fähigkeit, entzündungsauslösende Signalstoffe zu induzieren, ermöglichen in kombinierter Anwendung mit spezifischen Tumorantigenen die Herstellung einer stark immunogenen Vakzine, die zunächst eine starke direkte Immunreaktion gegen das Virus auslöst. Die im Gefolge dieser Immunreaktion auftretende Aktivierung etwa von Makrophagen, Killerzellen und T-Lymphozyten, sowie die dadurch induzierte Ausschüttung von Lymphokinen führt dann letztlich zu einer indirekten Aktivierung von tumorspezifischen T-Killerzellen, für die ansonsten die geringe Antigenität der nativen Tumorantigene nicht ausgereicht hätte. Diese zelluläre Immunreaktion gegen tumorspezifische Antigene kann dann therapeutisch genutzt werden, um das Anwachsen von Tumorzellen, bzw. das Auswachsen bereits bestehender Mikrometastasen nach Entfernung des Primärtumors zu verhindern.

Zur Herstellung der virusmodifizierten Tumorvakzine wird natives NDV unter sterilen Bedingungen für eine Stunde mit durch vorherige Bestrahlung inaktivierten Tumorzellen inkubiert. Daraus resultieren Tumorzellen, die an der Oberfläche das Virus adsorbiert tragen. Die Adsorption des NDV an die Tumorzellen kann an Stichproben fixierter Zellen durch ELISA mit anti-NDV-Antikörper verfolgt werden. Die Zellen werden dann in flüssigem Stickstoff bis zur Verwendung kryopräserviert.

Zur Verwendung als Vakzine werden die NDV-modifizierten Tumorzellen aufgetaut, ihre Vitalität wird bestimmt und es wird zur Inaktivierung des Virus bestrahlt. Die erste Vakzinierung erfolgt zweckmäßig 10 Tage postoperativ. Die Vakzinierung wird in wöchentlichem Abstand zwei bis dreimal wiederholt. Der Verlauf der Therapie wird anhand verschiedener Tests kontrolliert. Daran schließt sich die Nachbeobachtung (verbunden mit den Tests) des Patienten nach dem allgemeinen Nachsorgerichtlinien klinischer Studien an.

Während des Verlaufs werden neben den allgemeinen klinischen Daten in erster Linie Änderungen des immunologischen Status des Patienten während und nach der Therapie gemessen. Als Vergleichswerte dienen Untersuchungen vor und nach der Operation und vor Beginn der Immuntherapie. Die Untersuchungen und Tests erstrecken sich auf die zelluläre und homorale Immunantwort. Der Erfolg der Impfung kann auch durch Bestimmung des recidivfreien Intervalls kontrolliert werden.

Bezüglich der Anwendung kann zusammenfassend festgestellt werden, daß die Applikation frühestens eine Woche nach der Operation in Abständen von einer Woche, vorzugsweise mindestens dreimal erfolgt. Als Vakzine werden zwei oder drei oder auch mehr Injektionen mit modifizierten bestrahlten Tumorzellen sowie, für den Test also die Bestimmung der DTH-Reaktion eine weitere Injektion mit bestrahlten nicht-modifizierten Tumorzellen, vorzugsweise in geringerer Dosis, vorabreicht.

Die besten Ergebnisse werden erzielt, wenn für die Induktionsperiode, also die ersten zwei, drei oder vier Injektionen autologes Material verwendet wird, also Matrial, das von dem Tumor stammt, der dem Patienten selbst entnommen wurde.

Für die Aufrechterhaltungs-Therapie, also für spätere Injektionen in größeren Zeitabständen, z.B. alle drei Monate, wobei diese Aufrechterhaltungs-Therapie unter Umständen über Jahre aufrechterhalten werden soll, kann auch allogenes Material verwendet werden, also Material, das aus entsprechenden Tumoren Dritter stammt.

Die folgenden Beispiele erläutern die Erfindung.

### Beispiel 1

### Herstellung von inaktivierten Colorectalen Carcinom-Zellen.

Autologe Tumorzellen von Colorectalen Carcinomen wurden direkt aus dem frischen Operationspräparat eines Patienten gewonnen. Die Zellen wurden durch Bestrahlung mit 3 x 10⁴ rad pro 1,3 x 10⁷ Tumorzellen inaktiviert, so daß eine Vermehrung der Zellen im Patienten nicht möglich ist. Diese Zellen wurden für die Herstellung einer Vakzine aus NDV-modifizierten Tumorzellen verwendet. Sie werden in flüssigem Stickstoff bis zur Verwendung kryopräserviert.

### Beispiel 2

### Herstellung einer Vakzine aus NDV-modifizierten Tumorzellen

Die separierten bestrahlten Tumorzellen (1,3 x 10⁷ Zellen) wurden unter sterilen Bedingungen mit 160 HU NDV in serumfreiem Medium inkubiert. In bestimmten Zeitabständen wurden Aliquote der Zellen entnommen und fixiert. Mit einem ELISA mit anti-NDV-Antikörper und einem Immunoperoxidase Nachweissystem wurde die Adsorption des Virus an die Zelloberfläche bestimmt. Nach einer Stude waren ausreichende Mengen Virus an der Zelloberfläche adsorbiert. Die Virus-modifizierten Zellen werden in flüssigem Stickstoff bis zur Verwendung kryopräserviert. Die Zellen wurden vor Verwendung zur Inaktivierung des Virus nochmals mit 10 000 rad bestrahlt.

### Beispiel 3

### Vakzinierung von Patienten mit NDV-modifizierter Vakzine

Patienten die 10 Tage zuvor an einem colorectalen Carcinom ohne Fernmetastasen operiert worden waren wurden geimpft. Die Patienten wurden vor der Operation einem Hauttest (Merieux) mit 6 Recall-Antigenen unterzogen, um ihre Immunlage zu dokumentieren. Es wurde insgesamt drei- oder viermal in einwöchigen Abständen mit NDV-modifizierten Tumorzellen intradermal geimpft. (NDV/Tumorzellen-Verhältnis 1:1)

Bei den Patienten wurde das Auftreten von positiven Hautreaktionen vom verzögerten Typ (DTH-Reaktionen) als Hinweis auf eine tumorspezifische Immunisierung beobachtet.

## Patentansprüche

1. Virus-modifizierte, tumorspezifische Vakzine, bestehend aus durch Bestrahlung inaktivierten entsprechenden Tumorzellen, inkubiert mit NDV-Virus unter sterilen Bedingungen in serumfreiem Medium, dadurch gekennzeichnet, daß die Tumorzellen vom Operationspräparat des später zu behandelnden Patienten stammen und durch mechanische und enzymatische Methoden isoliert worden sind.

2. Vakzine nach Anspruch 1, dadurch gekennzeichnet, daß sie vor Applikation nochmals bestrahlt wird.

3. Verfahren zur Herstellung der Vakzine nach Anspruch 1 oder 2, durchgeführt außerhalb des Körpers, dadurch gekennzeichnet, daß man separierte, durch Bestrahlung inaktivierte entsprechende Tumorzellen, die vom Operationspräparat des später zu behandelnden Patienten stammen und durch mechanische und enzymatische Methoden isoliert sind, mit NDV-Virus unter sterilen Bedingungen in serumfreiem Medium inkubiert und die Adsorption des Virus durch ELISA an fixierten Zellen kontrolliert.

4. Verfahren nach Anspruch 3, dadurch gekennzeichnet, daß die Vakzine vor der Applikation durch neuerliche Bestrahlung nachsterilisiert wird.

5. Verfahren nach Anspruch 3 oder 4, dadurch gekennzeichnet, daß die Vakzine zwischen Inkubation und Applikation unter kryogenen Bedingungen, z.B. durch Kühlung mit festem Kohlendioxid oder flüssigem Stickstoff, aufbewahrt wird.

6. Impf- oder Injektionspräparat mit einem Gehalt an einer wirksamen Menge der Vakzine nach Anspruch 1 oder 2.

## Claims

1. Virus-modified, tumor-specific vaccines, consisting of corresponding tumor cells inactivated by radiation and incubated with NDV virus under sterile conditions in serum-free medium, characterized in that the tumor cells originate from surgical specimen from the patient to be treated subsequently and were isolated by mechanical and enzymatic methods.

2. Vaccines according to claim 1, characterized in that they are irradiated again prior to application.

3. Method for the preparation of the vaccines according to claim 1 or 2, carried out outside the body, characterized by incubating corresponding separated, radiation-inactivated tumor cells, originating from the surgical specimen from the patient to be treated subsequently and isolated by mechanical and enzymatic methods, with NDV virus under sterile conditions in serum-free medium and controlling the adsorption of the virus by ELISA on fixed cells.

4. Method according to claim 3, characterized in that the vaccines are resterilized by irradiating them once more prior to application.

5. The method according to claim 3 or 4, characterized in that the vaccines are stored between incubation and application under cryogenic conditions, e.g. by cooling with solid carbon dioxide or liquid nitrogen.

6. Inoculation or injection preparation containing an active amount of the vaccines according to claim 1 or 2.

## Revendications

1. Vaccin spécifique des tumeurs, modifié avec un virus, formé de cellules tumorales adéquates inactivées par irradiation, incubées avec le virus NDV (virus de la maladie de Newcastle) dans des conditions stériles dans un milieu ne contenant pas de sérum, caractérisé en ce que les cellules tumorales proviennent d'une préparation postopératoire du patient à traiter ultérieurement et ont été isolées par des procédés mécaniques et enzymatiques.

2. Vaccin selon la revendication 1, caractérisé en ce qu'on l'irradie de nouveau avant de l'administrer.

3. Procédé de préparation du vaccin selon la revendication 1 ou 2, effectué a l'extérieur du corps, caractérisé en ce qu'on fait incuber des cellules tumorales adéquates, séparées, inactivées par irradiation, qui proviennent d'une préparation postopératoire du patient à traiter ultérieurement et sont isolées par des procédés mécaniques et enzymatiques, avec le virus NDV dans des conditions stériles, dans un milieu ne contenant pas de sérum, et on maîtrise l'adsorption du virus sur des cellules fixées, par le procédé ELISA.

4. Procédé selon la revendication 3, caractérisé en ce qu'on stérilise le vaccin une nouvelle fois, de nouveau par irradiation, avant de l'administrer.

5. Procédé selon la revendication 3 ou 4, caractérisé en ce qu'on conserve le vaccin dans des conditions cryogènes entre l'incubation et l'administration, par exemple par refroidissement avec du dioxyde de carbone solide ou de l'azote liquide.

6. Préparation pour vaccination ou injection, contenant une quantité efficace du vaccin selon la revendication 1 ou 2.
